# EUROPEAN PATENT SPECIFICATION

(11) **EP 4 051 662 B1**
(45) Date of publication and mention of the grant of the patent: **07.02.2024**
(21) Application number: 20800823.5
(22) Date of filing: 29.10.2020
(51) Int. Cl.: C07D 209/42, C07D 401/12, C07D 471/04

(54) **SYNTHESIS OF 3-({5-CHLORO-1-[3-(METHYLSULFONYL)PROPYL]-1H-INDOL-2 YL}METHYL)-1-(2,2,2-TRIFLUOROETHYL)-1,3-DIHYDRO-2H-IMIDAZO[4,5-C]PYRIDIN-2-ONE**
SYNTHESE VON 3-({5-CHLOR-1-[3-(METHYLSULFONYL)PROPYL]-1-INDOL-2 YL} METHYL)-1-(2,2,2-TRIFLUOROETHYL)-1,3-DIHYDRO-2H-IMIDAZO[4,5-C]PYRIDIN-2-ON
SYNTHÈSE DE 3-({5-CHLORO-1-[3-(MÉTHYLSULFONYL)PROPYL]-1H-INDOL-2 YL} MÉTHYL)-1-(2,2,2-TRIFLUOROÉTHYL)-1,3-DIHYDRO-2H-IMIDAZO[4,5-C]PYRIDIN-2-ONE

(30) Priority: 30.10.2019 WO PCT/CN2019/114254
(43) Date of publication of application: 07.09.2022
(73) Proprietor: Janssen Sciences Ireland Unlimited Company, Ringaskiddy, Co Cork (IE)
(72) Inventor: RASPARINI, Marcello, 2340 Beerse (BE); WEERTS, Johan, Erwin, Edmond, 2340 Beerse (BE); JANSEN, Corina, Mathilde, 2340 Beerse (BE); LU, Zhihui, Shanghai (CN); TAN, Hongyu, Shanghai (CN); HAN, Licheng, Shanghai (CN)
(74) Representative: Verberckmoes, Filip Gerard
(86) International application number: PCT/EP2020/080381
(87) International publication number: WO 2021/083998

(56) References cited:
- WO-A1-2012/080447
- WO-A1-2013/186333

## Description

The present invention relates to a chemical synthesis route for preparing the RSV inhibiting compound 3-({5-chloro-1-[3-(methylsulfonyl)propyl]-1*H*-indol-2-yl}methyl)-1-(2,2,2-trifluoroethyl)-1,3-dihydro-2*H*-imidazo[4,5-c]pyridin-2-one, and to new compounds used as intermediate compounds in the multistep process.

### Background

Respiratory syncytial virus is a major cause of acute lower respiratory tract infection in young children, immunocompromised adults, and the elderly. Intervention with small-molecule antivirals specific for respiratory syncytial virus presents an important therapeutic opportunity, but no such compound is approved today.

Compound (1), *i.e.* 3-({5-chloro-1-[3-(methylsulfonyl)propyl]-1*H*-indol-2-yl}methyl)-1-(2,2,2-trifluoroethyl)-1,3-dihydro-2*H*-imidazo[4,5-c]pyridin-2-one, which can represented by the following structure : 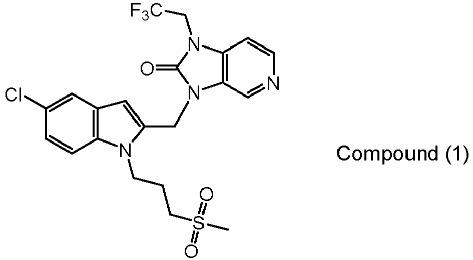 inhibits the replication of the respiratory syncytial virus (RSV) and has been described in WO-2012/080447 as compound P55.

### Prior art

The general pathway for the synthesis of compounds disclosed in WO-2012/080447 involves a Mitsunobu reaction between 2-hydroxymethyl substituted indoles and N-substituted 2-oxo-imidazopyridines. The Mitsunobu reaction, while very useful in small scale laboratory preparations, entails reagents (typically an excess of diisopropyl azodicarboxylate and of triphenylphosphine) that are not preferable in an industrial process. Those reagents generate stoichiometric amounts of diisopropylhydrazodicarboxylate and triphenylphosphine oxide as by-products, or similar by-products if alternative azodicarboxylates and phosphines are used. Removal of those by-products requires elaborate purifications via chromatography or multiple recrystallizations or re-slurries. Those purification operations are not desirable on large scale as they increase the cost of the final product by consuming solvents and purification aids (e.g., silica gel) and also decrease the yield of the desired product. Furthermore, the extended processing time in the plant to remove the reaction by-products, as well as the cost of their disposal, greatly reduce the usefulness of the Mitsunobu reaction at industrial production scale.

The general pathway for the synthesis of compounds disclosed in WO-2012/080447 can be found in Scheme 1 on page 11 and is depicted below :

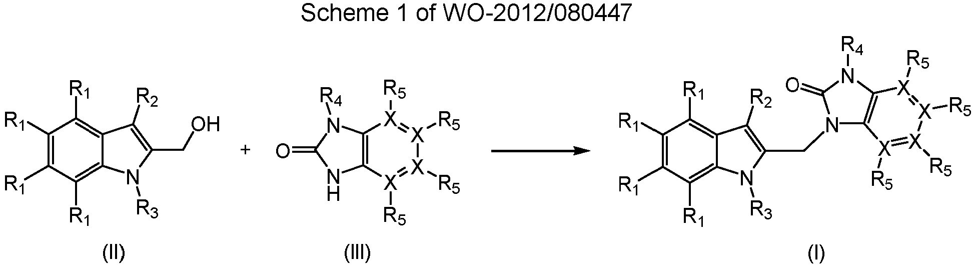

The coupling reaction as used in WO-2012/080447 to make the compounds of formula (I) of WO-2012/080447 is done according to Mitsunobu reaction conditions using diisopropyl azodicarboxylate and triphenyl phosphine in a suitable solvent such as DMF or THF. Use of the Mitsunobu route results in high levels of impurities that cannot easily be purged from the final compound by crystallization, preventing this process to be considered for commercial manufacturing. The use of the Mitsunobu reaction also results in the isolation of Compound (1) as a grey solid which is not desirable. The discoloration needs to be removed by chromatography, a purification technique that is not preferred for large scale production.

Hence, there is a need for an improved general synthesis route to obtain Compound (1) in high yield and with excellent purity that avoids the use of the Mitsunobu conditions at industrial scale. The process described herein affords highly pure, crystalline intermediates and Compound (1) with purity suitable for manufacturing the drug product. Each chemical step of this novel process is high yielding, uses cheap, safe and commercially available reagents, has a high atom-economy, and allows very efficient purification by crystallization from the reaction mixture.

### Description of the invention

A novel three-step process for preparing Compound (1) has been found that comprises the steps of amide formation, carbonyl reduction, and cyclization without the need to use Mitsunobu reaction conditions.

In a first embodiment, the present invention relates to a process for preparing Compound (1) 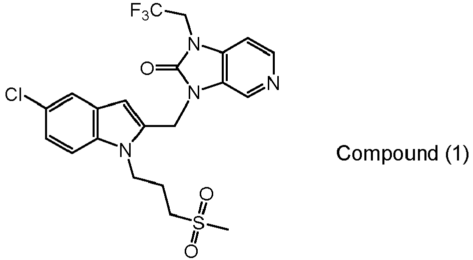 comprising the consecutive steps of
a) reacting 5-chloro-1-(3-(methylsulfonyl)propyl)-1/7-indole-2-carboxylic acid of formula (a), or a reactive functional derivative thereof, 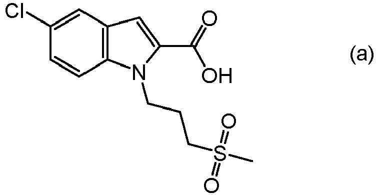 with *N*⁴-(2,2,2-trifluoroethyl)pyridine-3,4-diamine of formula (b) 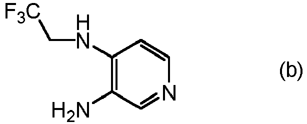 in an appropriate solvent in the presence of a coupling agent and optionally a base;
   to obtain 5-chloro-1-(3-(methylsulfonyl)propyl)-*N*-(4-((2,2,2-trifluoroethyl)amino)pyridin-3-yl)-1H-indole-2-carboxamide of formula (c); 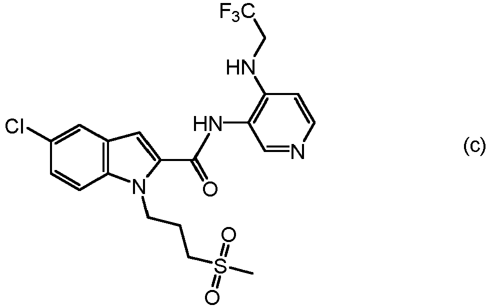
b) reducing the carbonyl group in compound (c) with a reducing agent to obtain *N*³-((5-chloro-1-(3-(methylsulfonyl)propyl)-1*H*-indol-2-yl)methyl)-*N*⁴-(2,2,2-trifluoroethyl)pyridine-3,4-diamine of formula (d); 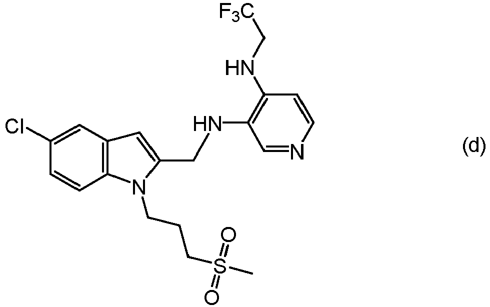
c) and reacting compound (d) in a suitable aprotic solvent with a carbonyl transfer reagent, optionally in the presence of an organic or inorganic base, to obtain Compound (1).

In a second embodiment the present invention concerns a novel compound of formula (a) 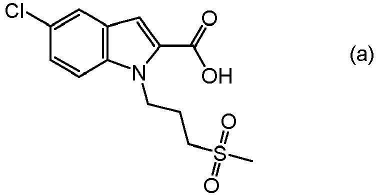 which is 5-chloro-1-(3-(methylsulfonyl)propyl)-1*H*-indole-2-carboxylic acid.

In a third embodiment the present invention concerns a novel compound of formula (c) 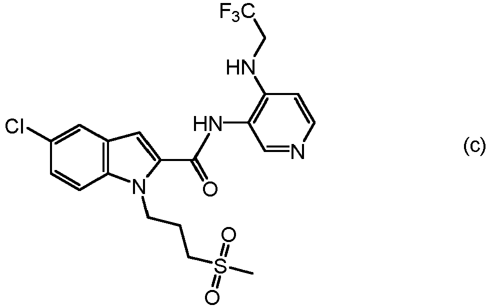 which is 5-chloro-1-(3-(methylsulfonyl)propyl)-*N*-(4-((2,2,2-trifluoroethyl)amino)pyridin-3-yl)-1*H-*indole-2-carboxamide.

In a fourth embodiment the present invention concerns a novel compound of formula (d) 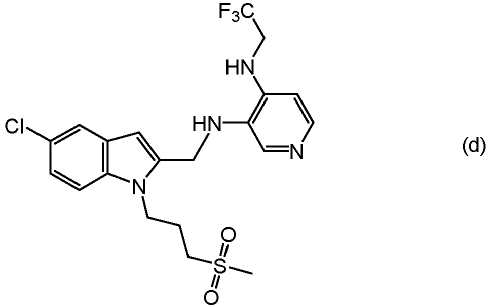 which is *N*³-((5-chloro-1-(3-(methylsulfonyl)propyl)-1*H*-indol-2-yl)methyl)-*N*⁴-(2,2,2-trifluoroethyl)pyridine-3,4-diamine.

Step a) is an art-known amide bond formation reaction between a carboxylic acid compound of formula (a) and an amine of formula (b) wherein said amide-bond formation can be performed by mixing the compounds (a) and (b) in an appropriate solvent in the presence of a coupling agent and optionally a base. Appropriate solvents are polar aprotic solvents such as, e.g., *N,N*-dimethyl formamide, *N,N*-dimethyl acetamide, N-methylpyrrolidone, *N*-butylpyrrolidone, tetrahydrofuran, 2-methyltetrahydrofuran, acetonitrile, propionitrile, and butyronitrile. The carboxylic acid compound of formula (a) can be used as such or can first be converted into an activated functional derivative thereof, e.g, acyl isourea, acyl imidazolide, acyl halide, or mixed anhydride. Convenient amide coupling agents are, e.g., EDC (*N*-(3-dimethylamino-propyl)-*N*'-ethylcarbodiimide hydrochloride), DIC (*N,N*'-diisopropylcarbodiimide), DCC (N,N'-dicyclohexylcarbodiimide), CDI (1,1'-carbonyldiimidazole), and T3P^{®} (1-propanephosphonic anhydride). Suitable optional bases for use in amide-bond formation reactions are, e.g., trialkyl amines (such as triethylamine, tributylamine, and *N,N*-diisopropylethylamine), cyclic amines (such as DBU (1,8-diaza-bicyclo[5.4.0]undec-7-ene) and DBN (1,5-diazabicyclo[4.3.0]non-5-ene)), pyridine type compounds (such as pyridine, 2- and 4- picoline, and 2,6-lutidine), and DMAP (4-dimethylamino-pyridine). The reaction may conveniently be carried out at a temperature ranging between room temperature and the reflux temperature of the reaction mixture. A highly desirable feature of the above described transformation is the high regioselectivity in the amide-forming step: no coupling is observed between the carboxylic acid of compound (a) and the secondary amine of compound (b), leading to increased yield and purity of compound (c).

Step b) is the conversion of the carbonyl group in the compound of formula (c) to a methylene group by treatment with an appropriate reducing agent. Appropriate reducing agents are, e.g., silicon hydrides (such as Et₃SiH and PMHS (poly(methylhydrosiloxane))), aluminium hydrides (such as Dibal-H (diisobutylaluminium hydride) and RedAI (sodium bis(2-methoxyethoxy)aluminium hydride)), and boron hydrides (such as LiBH₄, NaBH₄, and borane complexes BH₃.THF, and BH₃.Me₂S). Borane complexes can conveniently be generated *in situ* from NaBH₄ and a Brønsted acid such as sulfuric acid or a Lewis acid such as boron trihalides (optionally as a complex with an ether solvent), aluminium trichloride, or iodine. Suitable solvents for the amide reduction reaction are ethers, e.g., tetrahydrofuran (THF) and 2-methyltetrahydrofuran (2-Me-THF).

In step c) the diamine compound (d) is converted into Compound (1) using a carbonyl transfer reagent such as, e.g., CDI, urea, phosgene, diphosgene, triphosgene, or a chloroformate such as ethyl or phenyl chloroformate; in a suitable aprotic solvent, e.g., ethyl acetate, acetonitrile, propionitrile, butyronitrile, tetrahydrofuran, 2-methyltetrahydrofuran, *N,N*-dimethyl acetamide, *N,N*-dimethyl formamide, or *N*-methylpyrrolidone, to provide Compound (1). Optionally, an organic base is added, e.g., triethylamine, tributylamine, *N,N*-diisopropylethylamine, cyclic amines (such as DBU (1,8-diaza-bicyclo[5.4.0]-undec-7-ene) or DBN (1,5-diazabicyclo[4.3.0]non-5-ene)), imidazoles (such as imidazole or *N*-methylimidazole), pyridines (such as pyridine, 2- or 4-picoline, or 2,6-lutidine), DMAP (4-dimethylaminopyridine), or an inorganic base such as potassium carbonate.

Compound (1) can optionally be further purified by art-known techniques such as column chromatography or crystallisation.

### Example 1 : synthesis of 3-(methylsulfonyl)propyl 4-methylbenzenesulfonate

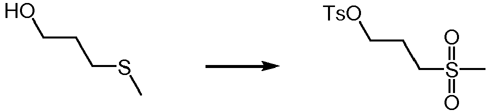

3-(Methylthio)propan-1-ol (100.00 g) is dissolved under nitrogen atmosphere in dichloromethane (500 mL) and tosyl chloride (188.50 g) is added. The solution is cooled to 0°C; *N,N,N',N'*-tetramethyl-1,6-hexanediamine (4.87 g) and triethylamine (114.40 g) are then added. After completion of the tosylation reaction, water is added and the mixture is stirred at a temperature of 10-15°C. After phase separation, the organic layer is washed with diluted aqueous HCl and subsequently with water. The organic layer is then added dropwise at 25°C to a vessel containing a solution of potassium peroxymonosulfate (Oxone^{™}) (752.60 g) in water (3000 mL) and stirred until complete oxidation. The organic layer is then washed with water; MTBE (1000 mL) is then added dropwise to the organic layer and, after complete addition, the mixture is cooled to 0°C. The solid is then filtered and dried under reduced pressure to give the product (246.70 g, 90% yield).

¹H NMR (600 MHz, CDCl₃) δ ppm 2.15 - 2.28 (m, 2 H); 2.45 (s, 3 H); 2.91 (s, 3 H); 3.05 - 3.17 (m, 2 H); 4.17 (t, J=5.95 Hz, 2 H); 7.37 (d, J=8.12 Hz, 2 H); 7.78 (d, J=8.31 Hz, 2 H) ¹³C NMR (151 MHz, CDCl₃) δ ppm 21.89; 22.45; 41.39; 51.06; 68.06; 128.22; 130.24; 132.72 ; 145.50

### Example 2 : synthesis of N⁴-(2,2,2-trifluoroethyl)pyridine-3,4-diamine

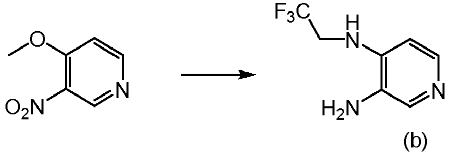

Citric acid monohoydrate (295.00 g) is dissolved in water (370.00 g) and 4-methoxy-3-nitropyridine (179.00 g) is added followed by 2,2,2-trifluoroethylamine (348.00 g). The mixture is stirred at 50°C until complete conversion. After cooling to room temperature, 2-methyltetrahydrofuran (1250 mL) is added and the phases are separated. The aqueous phase is reextracted with 2-methyltetrahydrofuran (530 mL). The combined organic layers are washed with 7% aqueous NaHCO₃ solution (890.00 g) and with water (903.00 g). The organic layer is concentrated to approximately 600 mL. Ethanol (1000 mL) is added, followed by Pd/C (10%, 50% wet, 7.50 g). The mixture is hydrogenated under 45-50 psi (310 - 345 kPa) hydrogen gas until complete conversion, then cooled to 30-40°C and filtered over diatomaceous earth (Celite^{®}), and the cake is washed with ethanol. The solvent is switched to pure 2-methyltetrahydrofuran by atmospheric distillation and parallel dosing of 2-methyltetrahydrofuran, reaching a volume of approximately 500 mL. The mixture is cooled to 50-55°C and toluene (1700 mL) is slowly added. After complete addition, the mixture is cooled to 0-5°C and the solid is then filtered and dried under reduced pressure to give product (b) (204.00 g, 92% yield).

¹H NMR (600 MHz, DMSO-*d*₆) δ ppm 4.03 (qd, J=9.50, 6.80 Hz, 2 H); 4.66 (br s, 2 H); 5.88 (t, J=6.80 Hz, 1 H); 6.60 (d, J=5.29 Hz, 1 H); 7.62 (d, J=5.29 Hz, 1 H); 7.71 (s, 1 H).

¹³C NMR (151 MHz, DMSO-*d*₆); δ ppm 43.08 (q, J=32.9 Hz); 104.68; 125.61 (q, J=281.0 Hz); 131.02; 135.30; 139.53; 139.94

### Example 3 : synthesis of 5-chloro-1-(3-(methylsulfonyl;propyl)-1H-indole-2-carboxylic acid

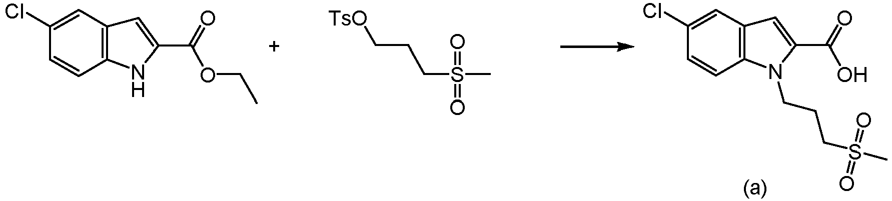

Ethyl-5-chloro-1/7-indole-2-carboxylate (50.00 g), 3-(methylsulfonyl)propyl-4-methylbenzenesulfonate (71.90 g), potassium carbonate (61.79 g), and tetrabutylammonium hydrogensulfate (3.79 g) are mixed in toluene (500 mL). The mixture is heated to 70°C until complete conversion, then water (250 mL) is slowly added and the phases are separated at 60°C. The water layer is discarded and water (100 mL) and 50% aqueous NaOH (23.25 g) are added; the mixture is stirred at 60°C until complete conversion. Water (150 mL) is added and the phases are separated at 60°C. A 80% portion (285.10 g) of the aqueous layer obtained (the other 20% was used for other purposes) is added in portions to a mixture of 34.5% w/w HCl (34.00 g), water (71 mL) and isopropanol (500 mL) at 50°C. Seeding material is added after 25% addition of the aqueous solution. The mixture is stirred for 4 hours at 50°C and then slowly cooled to 15°C. The product is filtered, washed with a mixture of water (36 mL) and isopropanol (36 mL) and dried under reduced pressure to obtain the product (a) (52.90 g, 93% yield).

¹H NMR (600 MHz, DMSO-*d*₆) δ ppm 2.07 - 2.18 (m, 2 H); 2.96 (s, 3 H); 3.07 - 3.14 (m, 2 H); 4.67 (t, J=7.18 Hz, 2 H); 7.24 (d, J=0.76 Hz, 1 H); 7.35 (dd, J=9.06, 2.27 Hz, 1 H); 7.70 (d, J=9.06 Hz, 1 H); 7.77 (d, J=2.20 Hz, 1 H).

¹³C NMR (151 MHz, DMSO-*d*₆) δ ppm 23.31; 39.99; 42.63; 50.92; 109.67; 112.63; 121.33; 124.83; 125.02; 126.39; 129.22; 137.03; 162.44.

### Example 4 : synthesis of 5-chloro-1-(3-(methylsulfonyl)propyl)-N-(4-((2,2,2-trifluoroethyl)amino)pyridin-3-yl)-1H-indole-2-carboxamide

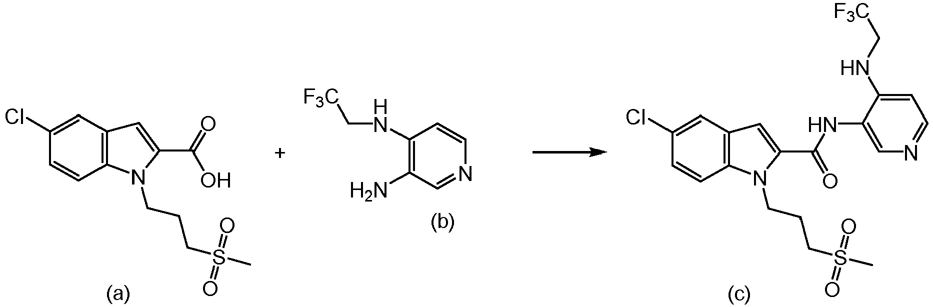

5-Chloro-1-(3-(methylsulfonyl)propyl)-1H-indole-2-carboxylic acid (= compound (a)) (25.00 g) and *N*⁴-(2,2,2-trifluoroethyl)pyridine-3,4-diamine (= compound (b)) (15.10 g) are mixed with DMAP (4-dimethyl-aminopyridine) (9.67 g) in acetonitrile (198 mL) at 20°C under nitrogen atmopshere. EDC (1-ethyl-3-(3-dimethylamino-propyl)carbodiimide hydrochloride) (30.35 g) is added and after 30 minutes at 20°C the mixture is gradually heated to 55°C. After additional 2 hours the mixture is gradually heated to 73°C and water (253 mL) is added over 1 hour. After 30 minutes, seeding material is added. The mixture is gradually cooled to 20°C, after 2 hours filtered and the cake is washed with a mixture of acetonitrile (24 mL) and water (40 mL) and dried under reduced pressure to obtain the product (c) (35.08 g, 91% yield).

¹H NMR (600 MHz, DMSO-*d*₆) ¹H NMR (600 MHz, DMSO-d6) δ ppm 2.13 - 2.23 (m, 2 H); 2.95 (s, 3 H); 3.08 - 3.19 (m, 2 H); 4.00 - 4.11 (m, 2 H) 4.65 (br t, J=7.18 Hz, 2 H); 6.66 (br t, J=6.61 Hz, 1 H); 6.89 (d, J=6.04 Hz, 1 H); 7.34 (dd, J=8.88, 2.08 Hz, 1 H); 7.44 (s, 1 H); 7.71 (d, J=8.69 Hz, 1 H); 7.82 (d, J=1.51 Hz, 1 H); 8.10 (s, 1 H); 8.13 (d, J=5.67 Hz, 1 H); 9.89 (s, 1 H).

¹³C NMR (151 MHz, DMSO-*d*₆) δ ppm 23.29; 39.99; 42.79; 42.88 (q, J= 33.2 Hz); 51.01; 105.81; 105.85; 112.33; 119.22; 120.90; 125.50 (q, J=282.1 Hz); 124.11; 124.96; 126.65; 132.42; 136.30; 148.16; 148.43; 149.23; 161.09 .

### Example 5 : synthesis of N³-((5-chloro-1-(3-(methylsulfonyl)propyl)-1H-indol-2-yl)methyl)-N⁴-(2,2,2-trifluoroethyl)pyridine-3,4-diamine

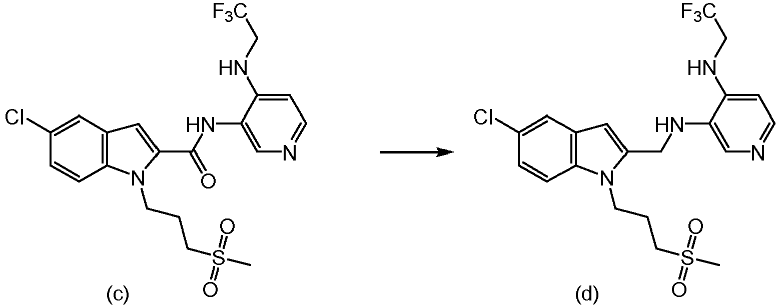

Compound (c) (45.00 g) is suspended in THF (450 mL) and the solvent is distilled with continuous addition of THF until the water content is <0.05% w/w. NaBH₄ (10.45 g) is then added followed by slow addition of BF₃.THF (51.51 g). Upon complete conversion, methanol (460 mL) is slowly dosed. The solvent is distilled and switched to pure 2-methyltetrahydrofuran (about 700 mL). Water (340 mL) and 50% aqueous NaOH (8.6 mL) are added. The mixture is stirred at 52°C for 16-24 hours, with continuous addition of 50% aqueous NaOH to keep the pH between 9.5 and 10.5. After phase separation the organic layer is washed at 52°C with water (368 mL). The organic phase is then azeotropically dried by distillation at atmospheric pressure and concentrated to a volume of about 300 mL. The mixture is gradually cooled to 15°C, after 8 hours filtered, washed with 2-methyltetrahydrofuran and dried under reduced pressure to obtain the product (d) (33.8 g, 77% yield).

¹H NMR (600 MHz, DMSO-*d*₆) δ ppm 2.12 (dd, J=7.60 Hz, 2 H); 2.94 (s, 3 H); 3.17 (t, J=7.81 Hz, 2 H); 4.01 - 4.10 (m, 2 H); 4.35 (t, J=7.45 Hz, 2 H); 4.51 (d, J=5.27 Hz, 2 H); 5.09 (t, J=5.18 Hz, 1 H); 6.07 (t, J=6.63 Hz, 1 H); 6.50 (s, 1 H); 6.67 (d, J=5.27 Hz, 1 H); 7.14 (dd, J=8.83, 1.91 Hz, 1 H); 7.55 (d, J=8.90 Hz, 1 H); 7.57 (d, J=2.00 Hz, 1 H); 7.73 (d, J=5.45 Hz, 1 H); 7.82 (s, 1 H).

¹³C NMR (151 MHz, DMSO-*d*₆) δ ppm 23.48; 40.35; 40.57; 41.97; 43.65 (q, J=32.7 Hz); 51.38; 101.45; 104.78; 111.68; 119.61; 121.41; 126.10 (q, J=282.3 Hz); 124.45; 128.83; 131.23; 132.55; 135.69; 139.75; 140.82; 141.24.

### Example 6 : synthesis of Compound (1)

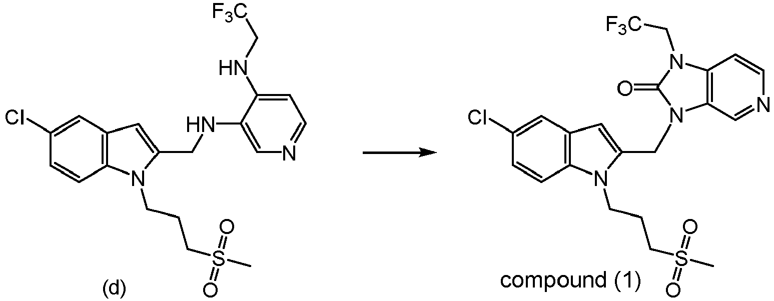

Compound (d) (38.00 g) and carbonyl diimidazole (26.00 g) are suspended in acetonitrile (480 mL) and the mixture is heated to 75°C until complete conversion. Acetonitrile (480 mL) is added , the mixture is cooled to 60°C, and seeding material is added followed by water (2.90 g). The mixture is concentrated by reduced pressure distillation at about 10°C to a final volume of about 350 mL. The slurry is filtered, washed with acetonitrile and dried under reduced pressure to obtain the product Compound (1) (36.00 g, 90% yield).

Compound (1) is further purified by crystallization from a mixture of 2-butanone and water (90;10 v/v, 10 volumes).

¹H NMR (500 MHz, DMSO-*d*₆) δ ppm 1.95 (m, 2 H); 2.98 (s, 3 H); 3.15 (t, J=7.90 Hz, 2 H); 4.38 (t, J=7.90 Hz, 2 H); 4.89 (q, J=9.30 Hz, 2 H); 5.40 (s, 2 H); 6.48 (s, 1 H); 7.17 (dd, J=8.70, 2.30 Hz, 1 H); 7.44 (d, J=5.30 Hz, 1 H); 7.55 (d, J=9.10 Hz, 1 H); 7.57 (d, J=1.90 Hz, 1 H); 8.31 (d, J=5.30 Hz, 1 H); 8.49 (s, 1H).

¹³C NMR (125 MHz, DMSO-*d*₆) δ ppm 22.95; 37.55; 41.60; 40.20; 42.07 (q, J=34.0 Hz); 50.82; 101.91; 104.59; 111.56; 119.56; 121.70; 124.32 (q, J=279.9 Hz); 124.31; 126.19; 128.04; 129.96; 134.98; 135.28; 135.57; 143.26; 152.44.

Melting point (DSC) : 216°C.

## Claims

1. A process for preparing 3-({5-chloro-1-[3-(methylsulfonyl)propyl]-1*H*-indol-2-yl}methyl)-1-(2,2,2-trifluoroethyl)-1,3-dihydro-2*H*-imidazo[4,5-c]pyridin-2-one
comprising the consecutive steps of
a) reacting 5-chloro-1-(3-(methylsulfonyl)propyl)-1*H*-indole-2-carboxylic acid of formula (a), or a reactive functional derivative thereof, 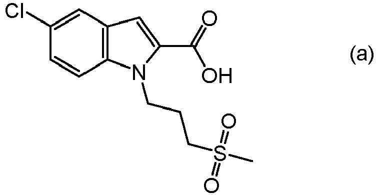 with *N*⁴-(2,2,2-trifluoroethyl)pyridine-3,4-diamine of formula (b) 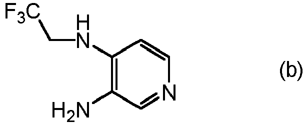 in an appropriate solvent in the presence of a coupling agent and optionally a base;
to obtain 5-chloro-1-(3-(methylsulfonyl)propyl)-*N*-(4-((2,2,2-trifluoroethyl)amino)pyridin-3-yl)-1*H*-indole-2-carboxamide of formula (c) 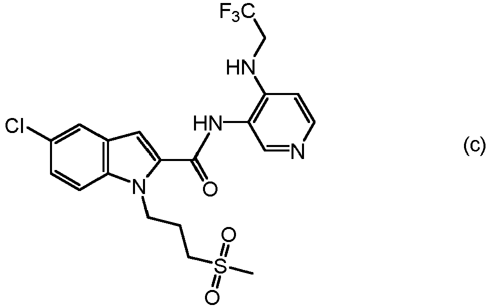
b) reducing the carbonyl group in compound (c) with a reducing agent to obtain *N*³-((5-chloro-1-(3-(methylsulfonyl)propyl)-1*H*-indol-2-yl)methyl)-*N*⁴-(2,2,2-trifluoroethyl)-pyridine-3,4-diamine of formula (d); 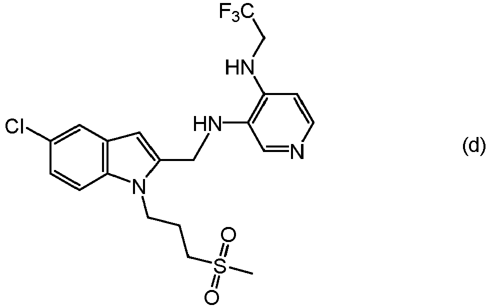
c) and reacting compound (d) in a suitable aprotic solvent with a carbonyl transfer reagent, optionally in the presence of an organic or inorganic base, to obtain 3-({5-chloro-1-[3-(methylsulfonyl)propyl]-1*H*-indol-2-yl}methyl)-1-(2,2,2-trifluoroethyl)-1,3-dihydro-2*H-*imidazo[4,5-c]pyridin-2-one.

2. The process as claimed in claim 1 wherein in step a) the appropriate solvent is a polar aprotic solvent.

3. The process as claimed in claim 2 wherein the polar aprotic solvent is selected from dimethyl formamide, *N,N*-dimethyl acetamide, *N*-methylpyrrolidone, *N*-butylpyrrolidone, tetrahydrofuran, 2-methyltetrahydrofuran, acetonitrile, propionitrile, and butyronitrile.

4. The process as claimed in claim 1 wherein in step a) the coupling agent is selected from EDC (*N*-(3-dimethylamino-propyl)-*N*'-ethylcarbodiimide hydrochloride), DIC (N,N'-diisopropylcarbodiimide), DCC (*N,N*'-dicyclohexylcarbodiimide), CDI (1,1'-carbonyldiimidazole), or T3P (1-propanephosphonic anhydride).

5. The process as claimed in claim 1 wherein in step a) the base is a trialkyl amine, a cyclic amine, or a pyridine like compound.

6. The process as claimed in claim 5 wherein the base is selected from triethylamine, tributylamine, *N,N*-diisopropylethylamine, DBU (1,8-diazabicyclo[5.4.0]undec-7-ene), DBN (1,5-diazabicyclo[4.3.0]non-5-ene), pyridine, 2- or 4- picoline, 2,6-lutidine, or DMAP (4-dimethylaminopyridine).

7. The process as claimed in claim 1 wherein in step b) the reducing agent is a boron or silicon or aluminium hydride or a borane complex.

8. The process as claimed in claim 7 wherein the reducing agent is selected from as Et₃SiH, poly(methylhydrosiloxane), diisobutylaluminium hydride, sodium bis(2-methoxy-ethoxy)-aluminium hydride), LiBH₄, NaBH₄, BH₃.THF, and BH₃.Me₂S.

9. The process as claimed in claim 1 wherein in step c) the carbonyl transfer reagent is selected from CDI, phosgene, triphosgene, ethyl chloroformate, or phenyl chloroformate.

10. The process as claimed in claim 1 wherein in step c) the suitable aprotic solvent is selected from ethyl acetate, acetonitrile, propionitrile, butyronitrile, tetrahydrofuran, 2-methyltetrahydrofuran, *N,N*-dimethyl acetamide, *N,N*-dimethyl formamide, or *N*-methylpyrrolidone.

11. The process as claimed in claim 10 wherein the optional organic or inorganic base is selected from triethylamine, tributylamine, *N,N*-diisopropylethylamine, DBU (1,8-diaza-bicyclo[5.4.0]undec-7-ene), DBN (1,5-diazabicyclo[4.3.0]non-5-ene), pyridine, 2- or 4-picoline or 2,6-lutidine or DMAP (4-dimethylaminopyridine), or potassium carbonate.

12. A compound of formula (a) 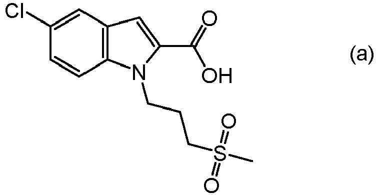 which is 5-chloro-1-(3-(methylsulfonyl)propyl)-1*H*-indole-2-carboxylic acid

13. A compound of formula (c) 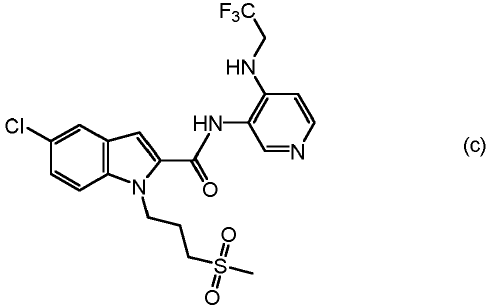 which is 5-chloro-l-(3-(methylsulfonyl)propyl)-*N*-(4-((2,2,2-trifluoroethyl)amino)pyridin-3-yl)-1*H*-indole-2-carboxamide.

14. A compound of formula (d) 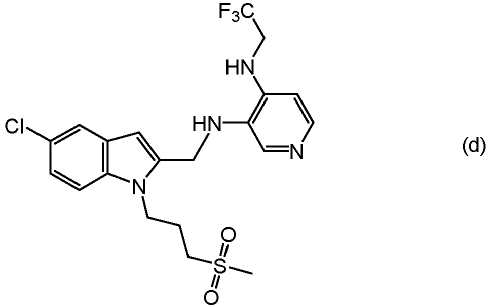 which is *N*³-((5-chloro-1-(3-(methylsulfonyl)propyl)-1*H*-indol-2-yl)methyl)-*N*⁴-(2,2,2-trifluoroethyl)pyridine-3,4-diamine

## Patentansprüche

1. Verfahren zum Herstellen von 3-({5-Chlor-1-[3-(methylsulfonyl)propyl]-1*H-*indol-2-yl}methyl)-1-(2,2,2-trifluorethyl)-1,3-dihydro-2H-imidazo[4,5-c]pyridin-2-on,
umfassend die aufeinanderfolgenden Schritte
a) Umsetzen von 5-Chlor-1-(3-(methylsulfonyl)propyl)-1*H*-indol-2-carbonsäure von Formel (a) oder einem reaktiven funktionellen Derivat davon, 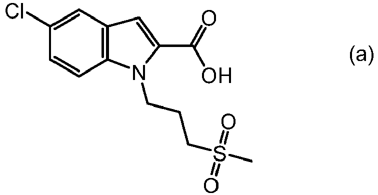 mit *N*⁴-(2,2,2-Trifluorethyl)pyridin-3,4-diamin von Formel (b) 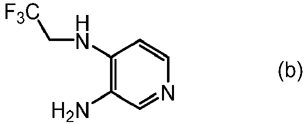 in einem geeigneten Lösungsmittel in der Gegenwart eines Haftvermittlers und optional einer Base;
um 5-Chlor-1-(3-(methylsulfonyl)propyl)-*N*-(4-((2,2,2-trifluorethyl)amino)pyridin-3-yl)-1H-indol-2-carboxamid von Formel (c) zu erhalten 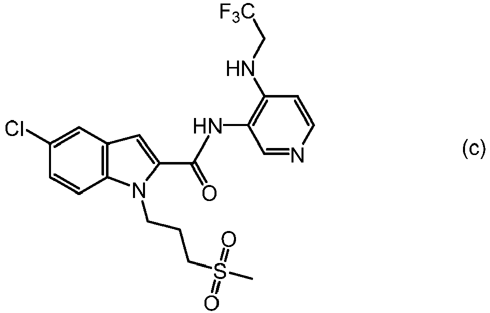
b) Reduzieren der Carbonylgruppe in Verbindung (c) mit einem Reduktionsmittel, um *N*³-((5-Chlor-1-(3-(methylsulfonyl)propyl)-1*H*-indol-2-yl)methyl)-*N*⁴-(2,2,2-trifluorethyl)pyridin-3,4-diamin von Formel (d) zu erhalten; 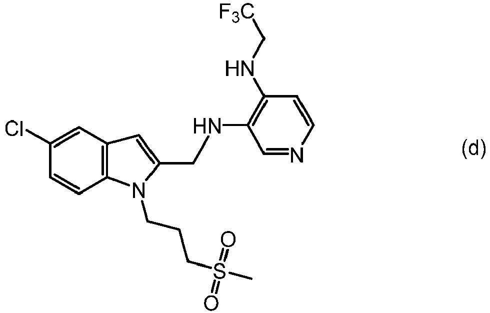
c) und Umsetzen von Verbindung (d) in einem passenden aprotischen Lösungsmittel mit einem Carbonyltransferreagens, optional in der Gegenwart einer organischen oder anorganischen Base, um 3-({5-Chlor-1-[3-(methylsulfonyl)propyl]-1*H*-indol-2-yl}methyl)-1-(2,2,2-trifluorethyl)-1,3-dihydro-2*H-*imidazo[4,5-c]pyridin-2-on zu erhalten.

2. Verfahren nach Anspruch 1, wobei in Schritt a) das geeignete Lösungsmittel ein polares aprotisches Lösungsmittel ist.

3. Verfahren nach Anspruch 2, wobei das polare aprotische Lösungsmittel aus Dimethylformamid, *N,N*-Dimethylacetamid, *N*-Methylpyrrolidon, *N-*Butylpyrrolidon, Tetrahydrofuran, 2-Methyltetrahydrofuran, Acetonitril, Propionitril und Butyronitril ausgewählt ist.

4. Verfahren nach Anspruch 1, wobei in Schritt a) der Haftvermittler aus EDC (*N*-(3-Dimethylaminopropyl)-*N*'-Ethylcarbodiimidhydrochlorid), DIC (N,N'-Diisopropylcarbodiimid), DCC (*N,N*'-Dicyclohexylcarbodiimid), CDI (1,1'-Carbonyldiimidazol) oder T3P (1-Propanphosphonsäureanhydrid) ausgewählt ist.

5. Verfahren nach Anspruch 1, wobei in Schritt a) die Base ein Trialkylamin, ein cyclisches Amin oder eine pyridinähnliche Verbindung ist.

6. Verfahren nach Anspruch 5, wobei die Base aus Triethylamin, Tributylamin, *N,N*-Diisopropylethylamin, DBU (1,8-Diazabicyclo[5.4.0]undec-7-en), DBN (1,5-Diazabicyclo[4.3.0]non-5-en), Pyridin, 2- oder 4-Picolin, 2,6-Lutidin oder DMAP (4-Dimethylaminopyridin) ausgewählt ist.

7. Verfahren nach Anspruch 1, wobei in Schritt b) das Reduktionsmittel ein Bor- oder Silizium- oder ein Aluminiumhydrid oder ein Borankomplex ist.

8. Verfahren nach Anspruch 7, wobei das Reduktionsmittel aus EtsSiH, Poly(methylhydrosiloxan), Diisobutylaluminiumhydrid, Natrium-bis-(2-methoxyethoxy)aluminiumhydrid), LiBH₄, NaBH₄, BH₃.THF und BH₃.Me₂S ausgewählt ist.

9. Verfahren nach Anspruch 1, wobei in Schritt c) das Carbonyltransferreagens aus CDI, Phosgen, Triphosgen, Chlorameisensäureethylester oder Chlorameisensäurephenylester ausgewählt ist.

10. Verfahren nach Anspruch 1, wobei in Schritt c) das passende aprotische Lösungsmittel aus Ethylacetat, Acetonitril, Propionitril, Butyronitril, Tetrahydrofuran, 2-Methyltetrahydrofuran, *N,N*-Dimethylacetamid, *N,N-*Dimethylformamid oder *N*-Methylpyrrolidon ausgewählt ist.

11. Verfahren nach Anspruch 10, wobei die optionale organische oder anorganische Base aus Triethylamin, Tributylamin, *N,N*-Diisopropylethylamin, DBU (1,8-Diazabicyclo[5.4.0]undec-7-en), DBN (1,5-Diazabicyclo[4.3.0]non-5-en), Pyridin, 2- oder 4-Picolin, 2,6-Lutidin oder DMAP (4-Dimethylaminopyridin) oder Kaliumcarbonat ausgewählt ist.

12. Verbindung von Formel (a), 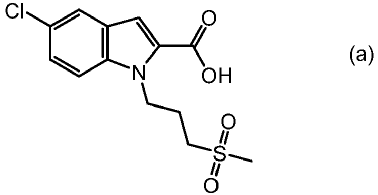 die 5-Chlor-1-(3-(methylsulfonyl)propyl)-1*H*-indol-2-carbonsäure ist

13. Verbindung von Formel (c), 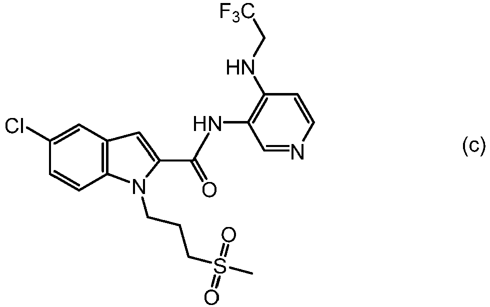 die 5-Chlor-1-(3-(methylsulfonyl)propyl)-*N*-(4-((2,2,2-trifluorethyl)amino)pyridin-3-yl)-1*H*-indol-2-carboxamid ist.

14. Verbindung von Formel (d), 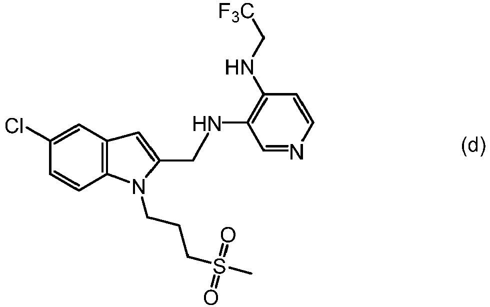 die *N*³-((5-Chlor-1-(3-(methylsulfonyl)propyl)-1*H*-indol-2-yl)methyl)-*N*⁴-(2,2,2-trifluorethyl)pyridin-3,4-diamin ist

## Revendications

1. Procédé de préparation de 3-({5-chloro-1-[3-(méthylsulfonyl)propyl]-1H-indol-2-yl}méthyl)-1-(2,2,2-trifluoroéthyl)-1,3-dihydro-2*H-*-imidazo[4,5-c]pyridin-2-one
comprenant les étapes consécutives consistant à
a) faire réagir de l'acide 5-chloro-1-(3-(méthylsulfonyl)propyl)-1H-indol-2-carboxylique de formule (a), ou un dérivé fonctionnel réactif de celui-ci, 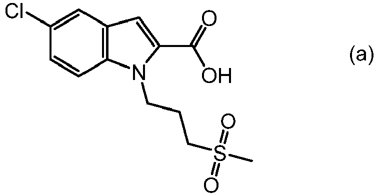 avec *N*⁴-(2,2,2-trifluoroéthyl)pyridin-3,4-diamine de formule (b) 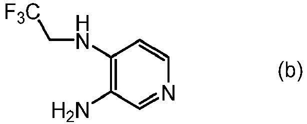 dans un solvant approprié en présence d'un agent de couplage et éventuellement d'une base ;
pour obtenir 5-chloro-1-(3-(méthylsulfonyl)propyl)-*N*-(4-((2,2,2-trifluoroéthyl)amino)pyridin-3-yl)-1H-indol-2-carboxamide de formule (c) 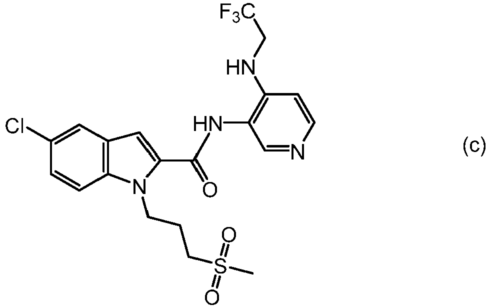
b) réduire le groupe carbonyle dans le composé (c) avec un agent réducteur pour obtenir *N*³-((5-chloro-1-(3-(méthylsulfonyl)propyl)-1*H*-indol-2-yl)méthyl)-*N*⁴-(2,2,2-trifluoroéthyl)-pyridin-3,4-diamine de formule (d) ; 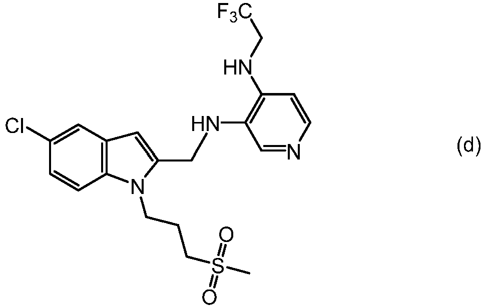
c) et faire réagir le composé (d) dans un solvant aprotique approprié avec un réactif de transfert de carbonyle, éventuellement en présence d'une base organique ou inorganique, pour obtenir du 3-({5-chloro-1-[3-(méthylsulfonyl)propyl]-1*H*-indol-2-yl}méthyl)-1-(2,2,2-trifluoroéthyl)-1,3-dihydro-2H-imidazo [4,5-c] pyridin-2-one.

2. Procédé selon la revendication 1, dans lequel à l'étape a) le solvant approprié est un solvant aprotique polaire.

3. Procédé selon la revendication 2, dans lequel le solvant aprotique polaire est choisi parmi diméthylformamide, *N,N*-diméthylacétamide, *N*-méthylpyrrolidone, *N*-butylpyrrolidone, tétrahydrofurane, 2-méthyltétrahydrofurane, acétonitrile, propionitrile et butyronitrile.

4. Procédé selon la revendication 1, dans lequel à l'étape a) l'agent de couplage est choisi parmi EDC (chlorhydrate de *N*-(3-diméthylamino-propyl)-*N-*éthylcarbodiimide), DIC (*N,N*'-diiso-propylcarbodiimide), DCC (*N,N*'dicyclohexylcarbodiimide), CDI (1,1'-carbonyldiimidazole), ou T3P (anhydride 1-propanephosphonique).

5. Procédé selon la revendication 1, dans lequel à l'étape a) la base est une trialkyl amine, une amine cyclique, ou un composé de type pyridine.

6. Procédé selon la revendication 5, dans lequel la base est choisie parmi triéthylamine, tributylamine, *N,N*-diisopropyléthylamine, DBU (1,8-diazabicyclo[5.4.0]undéc-7-ène), DBN (1,5-diazabicyclo[4.3.0]non-5-ène), pyridine, 2- ou 4- picoline, 2,6-lutidine, ou DMAP (4-diméthylaminopyridine).

7. Procédé selon la revendication 1 dans lequel à l'étape b) l'agent réducteur est un hydrure de bore, de silicium ou d'aluminium ou un complexe de borane.

8. Procédé selon la revendication 7, dans lequel l'agent réducteur est choisi parmi EtsSiH, poly(méthylhydrosiloxane), hydrure de diisobutylaluminium, bis(2-méthoxy-éthoxy)aluminohydrure) de sodium, LiBH₄, NaBH₄, BH₃.THF, et BH₃.Me₂S.

9. Procédé selon la revendication 1, dans lequel à l'étape c) le réactif de transfert de carbonyle est choisi parmi CDI, phosgène, triphosgène, chloroformiate d'éthyle ou chloroformiate de phényle.

10. Procédé selon la revendication 1, dans lequel à l'étape c) le solvant aprotique approprié est choisi parmi acétate d'éthyle, acétonitrile, propionitrile, butyronitrile, tétrahydrofurane, 2-méthyl-tétrahydrofurane, *N,N*-diméthylacétamide, *N,N*-diméthylformamide, ou *N*-méthylpyrrolidone.

11. Procédé selon la revendication 10, dans lequel la base organique ou inorganique éventuelle est choisie parmi triéthylamine, tributylamine, *N,N*-diisopropyléthylamine, DBU (1,8-diaza-bicyclo[5.4.0]undéc-7-ène), le DBN (1,5-diazabicyclo[4.3.0]non-5-ène), pyridine, 2- ou 4-picoline ou 2,6-lutidine ou DMAP (4-diméthylaminopyridine), ou carbonate de potassium.

12. Composé de formule (a) 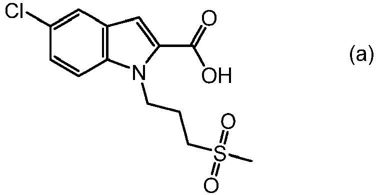 qui est acide 5-chloro-1-(3-(méthylsulfonyl)propyl)-1H-indol-2-carboxylique

13. Composé de formule (c) 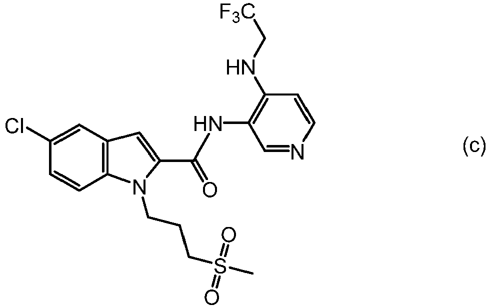 qui est 5-chloro-1-(3-(méthylsulfonyl)propyl)-*N*-(4-((2,2,2-trifluoroéthyl)amino)pyridin-3-yl)-1*H*-indol-2-carboxamide.

14. Composé de formule (d) 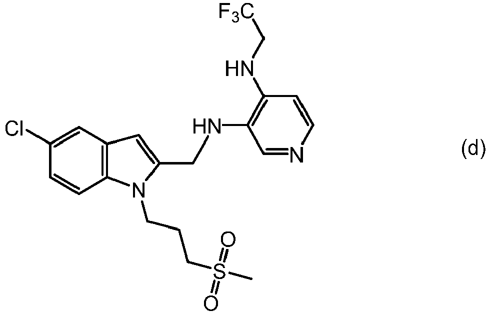 qui est *N*³-((5-chloro-1-(3-(méthylsulfonyl)propyl)-1*H*-indol-2-yl)méthyl)-*N*⁴-(2,2,2-trifluoroéthyl)pyridin-3,4-diamine
